# EUROPEAN PATENT APPLICATION

(11) **EP 4 789 605 A2**
(43) Date of publication of application: **12.08.2026**
(21) Application number: 26187849.0
(22) Date of filing: 03.12.2019
(51) Int. Cl.: A61B 5/00

(54) **EXTENDED WEAR AMBULATORY ELECTROCARDIOGRAPHY AND PHYSIOLOGICAL SENSOR MONITOR**

(30) Priority: 03.12.2018 US 201816208468
(62) Document number(s) of the earlier application(s) in accordance with Art. 76 EPC: 19828053.9 / 3 890 590
(71) Applicant: Bardy Diagnostics, Inc., Seattle, WA 98104 (US)
(72) Inventor: FELIX, Jason, Vashon Island, 98070 (US); BARDY, Gust H., Carnation, 98014 (US); BISHAY, Jon M., Lexington, 40502 (US); GREEN, Joshua D., Seattle, 98102 (US)
(74) Representative: Murgitroyd & Company

(57) **Abstract**

A moisture-resistant electrocardiography monitor, comprising: an electrocardiography monitor recorder, comprising: a wearable housing molded out of one or more materials and sealed against moisture; a plurality of electrical contacts protruding from the wearable housing; a seal coupling positioned on the wearable housing and surrounding the electrical contacts; and electronic circuitry provided within the wearable housing, the electronic circuitry further comprising: an electrocardiographic front end circuit under a control of a micro-controller and adapted to sense cardiac electrical potential differentials through electrocardiographic electrodes via some of the electrical contacts, which are provided to the micro-controller as an analog signal; the micro-controller configured to sample the analog signal; and a memory electrically interfaced with the micro-controller and operable to store the samples; and an extended wear electrode patch, comprising: a flexible backing comprising a plurality of adhesive contact surfaces; the electrocardiographic electrodes, each comprised on one of the adhesive contact surfaces; a receptacle affixed to a non-contacting surface of the flexible backing into which the wearable housing can be removably secured, the receptacle comprising a compartment within which a component interfaced to the electronic circuitry is positioned via some of the electrical contacts, wherein the component is a battery; a plurality of electrical pads positioned on the receptacle, each of the pads configured to interface with one of the electrical contacts when the wearable housing is secured within the receptacle; and a moisture-resistant seal formed on the receptacle and surrounding the electrical pads, wherein the moisture-resistant seal mates the seal coupling when the wearable housing is secured within the receptacle.

## Description

### TECHNICAL FIELD

This application relates in general to electrocardiographic monitoring and, in particular, to an extended wear ambulatory electrocardiography and physiological sensor monitor.

### BACKGROUND ART

The heart emits electrical signals as a by-product of the propagation of the action potentials that trigger depolarization of heart fibers. An electrocardiogram (ECG) measures and records such electrical potentials to visually depict the electrical activity of the heart over time. Conventionally, a standardized set format 12-lead configuration is used by an ECG machine to record cardiac electrical signals from well-established traditional chest locations. Electrodes at the end of each lead are placed on the skin over the anterior thoracic region of the patient's body to the lower right and to the lower left of the sternum, on the left anterior chest, and on the limbs. Sensed cardiac electrical activity is represented by PQRSTU waveforms that can be interpreted post-ECG recordation to derive heart rate and physiology. The P-wave represents atrial electrical activity. The QRSTU components represent ventricular electrical activity.

An ECG is a tool used by physicians to diagnose heart problems and other potential health concerns. An ECG is a snapshot of heart function, typically recorded over 12 seconds, that can help diagnose rate and regularity of heartbeats, effect of drugs or cardiac devices, including pacemakers and implantable cardioverter-defibrillators (ICDs), and whether a patient has heart disease. ECGs are used in-clinic during appointments, and, as a result, are limited to recording only those heart-related aspects present at the time of recording. Sporadic conditions that may not show up during a spot ECG recording require other means to diagnose them. These disorders include fainting or syncope; rhythm disorders, such as tachyarrhythmias and bradyarrhythmias; apneic episodes; and other cardiac and related disorders. Thus, an ECG only provides a partial picture and can be insufficient for complete patient diagnosis of many cardiac disorders.

Diagnostic efficacy can be improved, when appropriate, through the use of long-term extended ECG monitoring. Recording sufficient ECG and related physiology over an extended period is challenging, and often essential to enabling a physician to identify events of potential concern. A 30-day observation period is considered the "gold standard" of ECG monitoring, yet achieving a 30-day observation day period has proven unworkable because such ECG monitoring systems are arduous to employ, cumbersome to the patient, and excessively costly. Ambulatory monitoring in-clinic is implausible and impracticable. Nevertheless, if a patient's ECG could be recorded in an ambulatory setting, thereby allowing the patient to engage in activities of daily living, the chances of acquiring meaningful information and capturing an abnormal event while the patient is engaged in normal activities becomes more likely to be achieved.

For instance, the long-term wear of ECG electrodes is complicated by skin irritation and the inability ECG electrodes to maintain continual skin contact after a day or two. Moreover, time, dirt, moisture, and other environmental contaminants, as well as perspiration, skin oil, and dead skin cells from the patient's body, can get between an ECG electrode, the non-conductive adhesive used to adhere the ECG electrode, and the skin's surface. All of these factors adversely affect electrode adhesion and the quality of cardiac signal recordings. Furthermore, the physical movements of the patient and their clothing impart various compressional, tensile, and torsional forces on the contact point of an ECG electrode, especially over long recording times, and an inflexibly fastened ECG electrode will be prone to becoming dislodged. Notwithstanding the cause of electrode dislodgment, depending upon the type of ECG monitor employed, precise re-placement of a dislodged ECG electrode maybe essential to ensuring signal capture at the same fidelity. Moreover, dislodgment may occur unbeknownst to the patient, making the ECG recordings worthless. Further, some patients may have skin that is susceptible to itching or irritation, and the wearing of ECG electrodes can aggravate such skin conditions. Thus, a patient may want or need to periodically remove or replace ECG electrodes during a long-term ECG monitoring period, whether to replace a dislodged electrode, reestablish better adhesion, alleviate itching or irritation, allow for cleansing of the skin, allow for showering and exercise, or for other purpose. Such replacement or slight alteration in electrode location actually facilitates the goal of recording the ECG signal for long periods of time; however, ensuring that the level of quality of ECG recording and patient service remains constant over an extended period of time is dependent upon the monitoring equipment being up to a known standard. Use of third party consumables, such as ECG electrodes, could undermine expectations of ECG recording fidelity and adversely skew monitoring results.

Conventionally, Holter monitors are widely used for long-term extended ECG monitoring. Typically, they are used for only 24-48 hours. A typical Holter monitor is a wearable and portable version of an ECG that include cables for each electrode placed on the skin and a separate battery-powered ECG recorder. The cable and electrode combination (or leads) are placed in the anterior thoracic region in a manner similar to what is done with an in-clinic standard ECG machine. The duration of a Holter monitoring recording depends on the sensing and storage capabilities of the monitor, as well as battery life. A "looping" Holter monitor (or event) can operate for a longer period of time by overwriting older ECG tracings, thence "recycling" storage in favor of extended operation, yet at the risk of losing event data. Although capable of extended ECG monitoring, Holter monitors are cumbersome, expensive and typically only available by medical prescription, which limits their usability. Further, the skill required to properly place the electrodes on the patient's chest hinders or precludes a patient from replacing or removing the precordial leads and usually involves moving the patient from the physician office to a specialized center within the hospital or clinic.

The ZIO XT Patch and ZIO Event Card devices, manufactured by iRhythm Tech., Inc., San Francisco, CA, are wearable stick-on monitoring devices that are typically worn on the upper left pectoral region to respectively provide continuous and looping ECG recording. The location is used to simulate surgically implanted monitors. Both of these devices are prescription-only and for single patient use. The ZIO XT Patch device is limited to a 14-day monitoring period, while the electrodes only of the ZIO Event Card device can be worn for up to 30 days. The ZIO XT Patch device combines both electronic recordation components, including battery, and physical electrodes into a unitary assembly that adheres to the patient's skin. The ZIO XT Patch device uses adhesive sufficiently strong to support the weight of both the monitor and the electrodes over an extended period of time and to resist disadherance from the patient's body, albeit at the cost of disallowing removal or relocation during the monitoring period. Moreover, throughout monitoring, the battery is continually depleted and battery capacity can potentially limit overall monitoring duration. The ZIO Event Card device is a form of downsized Holter monitor with a recorder component that must be removed temporarily during baths or other activities that could damage the non-waterproof electronics. Both devices represent compromises between length of wear and quality of ECG monitoring, especially with respect to ease of long term use, female-friendly fit, and quality of atrial (P-wave) signals. Moreover, both devices rely on the same set of ECG electrodes for the duration of the monitoring period; signal capture can suffer as the ECG electrodes disadhere from the patient's body over time.

Therefore, a need remains for an extended wear continuously recording ECG monitor practicably capable of being worn for a long period of time in both men and women and capable of recording atrial signals reliably with quality assurance implemented as part of disposable component replenishment.

A further need remains for a device capable of recording signals ideal for arrhythmia discrimination, especially a device designed for atrial activity recording.

### DISCLOSURE OF THE INVENTION

Physiological monitoring can be provided through a wearable monitor that includes two components, a flexible extended wear electrode patch and a removable reusable monitor recorder. The wearable monitor sits centrally (in the midline) on the patient's chest along the sternum oriented top-to-bottom. The placement of the wearable monitor in a location at the sternal midline (or immediately to either side of the sternum), with its unique narrow "hourglass"-like shape, benefits long-term extended wear by removing the requirement that ECG electrodes be continually placed in the same spots on the skin throughout the monitoring period. Instead, the patient is free to place an electrode patch anywhere within the general region of the sternum, the area most likely to record high quality atrial signals or P-waves. In addition, ensuring that the level of quality of ECG recording and patient service remains constant over an extended period of time is provided through self-authentication of electrode patches (and other accessories). The monitor recorder implements a challenge response scheme upon being connected to an electrode patch (or other accessory). Failing self-authentication, the monitor recorder signals an error condition. In addition, electrode patches (and other accessories) can be limited to operating for only a certain period of time, or with pre-defined operational parameters or privileges. In a further embodiment, a patient can purchase additional time, pre-defined operational parameters or privileges through a server-based subscription service.

One embodiment provides an extended wear electrocardiography and physiological sensor monitor recorder. A sealed housing is adapted to be removably secured into the non-conductive receptacle on a disposable extended wear electrode patch. Electronic circuitry is included within the sealed housing. An externally-powered micro-controller is operable to execute under micro programmable control only upon authentication of the disposable extended wear electrode patch during power up of the electronic circuitry. An electrocardiographic front end circuit is electrically interfaced to the micro-controller and is operable to sense electrocardiographic signals through electrocardiographic electrodes provided on the disposable extended wear electrode patch. Externally-powered flash memory is electrically interfaced with the micro-controller and is operable to store samples of the electrocardiographic signals.

A further embodiment provides an extended wear electrocardiography patch. A flexible backing includes an elongated strip of stretchable material. An electrocardiographic electrode is respectively affixed to and conductively exposed on each end of the elongated strip. A flexible circuit is affixed on each end to the elongated strip and includes a pair of circuit traces each originating within one of the ends of the elongated strip and coupled to one of the electrocardiographic electrodes. A non-conductive receptacle securely adhered on the one end of the elongated strip and includes electrode terminals aligned to interface the pair of circuit traces to an electrocardiography monitor to obtain electrocardiographic signals through the electrocardiographic electrodes. A crypto circuit includes memory programed with a sampling rate for at least one physiological sensor provided at least one of with the electrocardiography monitor and on the flexible backing to instruct the physiological sensor to obtain readings of physiological data.

A still further embodiment provides an extended wear electrocardiography and physiological sensor monitor. An electrocardiography monitor includes a sealed housing and electronic circuitry within the sealed housing, including an externally-powered micro-controller and an electrocardiographic front end circuit electrically interfaced to the micro-controller and operable to sense electrocardiographic signals. An electrocardiography patch includes a flexible backing formed of an elongated strip of stretchable material and an electrocardiographic electrode affixed to and conductively exposed on a contact surface of each end of the elongated strip. A flexible circuit is affixed on each end to the elongated strip and includes a pair of circuit traces each originating within one of the ends of the elongated strip and electrically coupled to one of the electrocardiographic electrodes. A non-conductive receptacle is securely adhered on the one end of the elongated strip opposite the contact surface and formed to removably receive the electrocardiography monitor. The non-conductive receptacle includes electrode terminals aligned to electrically interface the pair of circuit traces to the electrocardiography monitor. A crypto circuit includes memory programed with a sampling rate for at least one physiological sensor provided at least one of with the electrocardiography monitor and on the flexible backing to instruct the physiological sensor to obtain readings of physiological data.

The monitoring patch is especially suited to the female anatomy. The narrow longitudinal midsection can fit nicely within the intermammary cleft of the breasts without inducing discomfort, whereas conventional patch electrodes are wide and, if adhesed between the breasts, would cause chafing, irritation, frustration, and annoyance, leading to low patient compliance.

The foregoing aspects enhance ECG monitoring performance and quality, facilitating long-term ECG recording, critical to accurate arrhythmia diagnosis.

In addition, the foregoing aspects enhance comfort in women (and certain men), but not irritation of the breasts, by placing the monitoring patch in the best location possible for optimizing the recording of cardiac signals from the atrium, another feature critical to proper arrhythmia diagnosis.

Finally, the foregoing aspects as relevant to monitoring are equally applicable to recording other physiological measures, such as temperature, respiratory rate, blood sugar, oxygen saturation, and blood pressure, as well as other measures of body chemistry and physiology.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, all without departing from their spirit and the scope. Accordingly, the drawings and detailed description are to be regarded as illustrative in nature and not as restrictive.

### DESCRIPTION OF THE DRAWINGS

FIGURES 1 and 2 are diagrams showing, by way of examples, an extended wear electrocardiography and physiological sensor monitor respectively fitted to the sternal region of a female patient and a male patient.
FIGURE 3 is a functional block diagram showing a system for providing a self-authenticating electrocardiography monitoring circuit in accordance with one embodiment.
FIGURE 4 is a perspective view showing an extended wear electrode patch with a monitor recorder in accordance with one embodiment inserted.
FIGURE 5 is a perspective view showing the monitor recorder of FIGURE 4.
FIGURE 6 is a perspective view showing the extended wear electrode patch of FIGURE 4 without a monitor recorder inserted.
FIGURE 7 is a bottom plan view of the monitor recorder of FIGURE 4
FIGURE 8 is a top view showing the flexible circuit of the extended wear electrode patch of FIGURE 4 when mounted above the flexible backing.
FIGURE 9 is an exploded view showing the component layers of the extended wear electrode patch of FIGURE 4.
FIGURE 10 is an alternative exploded view showing the component layers of the extended wear electrode patch of FIGURE 4.
FIGURE 11 is an exploded view showing an integrated flex circuit of the extended wear electrode patch of FIGURE 10.
FIGURE 12 is a functional block diagram showing the component architecture of the circuitry of the monitor recorder of FIGURE 4.
FIGURE 13 is a functional block diagram showing the circuitry of the extended wear electrode patch of FIGURE 4.
FIGURE 14 is a flow diagram showing a monitor recorder-implemented method for monitoring ECG data for use in the monitor recorder of FIGURE 4
FIGURE 15 is a graph showing, by way of example, a typical ECG waveform.
FIGURE 16 is a flow diagram showing a method for offloading and converting ECG and other physiological data from an extended wear electrocardiography and physiological sensor monitor in accordance with one embodiment.
FIGURE 17 is a flow diagram showing a method for providing a self-authenticating electrocardiography monitoring circuit in accordance with one embodiment
FIGURE 18 is a flow diagram showing, by way of example, a process for obtaining physiological data from a physiological sensor.

### BEST MODE FOR CARRYING OUT THE INVENTION

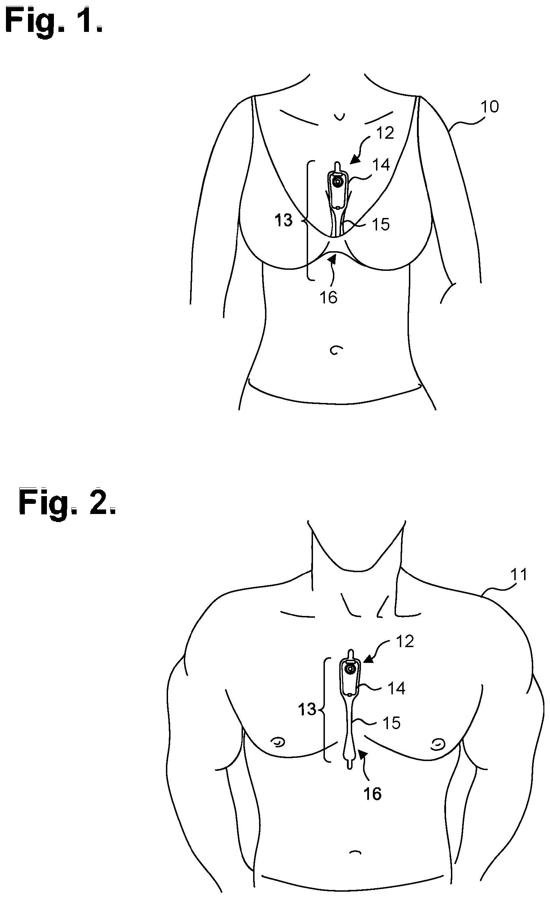

Physiological monitoring can be provided through a wearable monitor that includes two components, a flexible extended wear electrode patch and a removable reusable monitor recorder. FIGURES 1 and 2 are diagrams showing, by way of examples, an extended wear electrocardiography and physiological sensor monitor 12, including a monitor recorder 14 in accordance with one embodiment, respectively fitted to the sternal region of a female patient 10 and a male patient 11. The wearable monitor 12 sits centrally (in the midline) on the patient's chest along the sternum 13 oriented top-to-bottom with the monitor recorder 14 preferably situated towards the patient's head. In a further embodiment, the orientation of the wearable monitor 12 can be corrected post-monitoring, as further described *infra.* The electrode patch 15 is shaped to fit comfortably and conformal to the contours of the patient's chest approximately centered on the sternal midline 16 (or immediately to either side of the sternum 13). The distal end of the electrode patch 15 extends towards the Xiphoid process and, depending upon the patient's build, may straddle the region over the Xiphoid process. The proximal end of the electrode patch 15, located under the monitor recorder 14, is below the manubrium and, depending upon patient's build, may straddle the region over the manubrium.

The placement of the wearable monitor 12 in a location at the sternal midline 16 (or immediately to either side of the sternum 13) significantly improves the ability of the wearable monitor 12 to cutaneously sense cardiac electric signals, particularly the P-wave (or atrial activity) and, to a lesser extent, the QRS interval signals in the ECG waveforms that indicate ventricular activity, while simultaneously facilitating comfortable long-term wear for many weeks. The sternum 13 overlies the right atrium of the heart and the placement of the wearable monitor 12 in the region of the sternal midline 13 puts the ECG electrodes of the electrode patch 15 in a location better adapted to sensing and recording P-wave signals than other placement locations, say, the upper left pectoral region or lateral thoracic region or the limb leads. In addition, placing the lower or inferior pole (ECG electrode) of the electrode patch 15 over (or near) the Xiphoid process facilitates sensing of ventricular activity and provides superior recordation of the QRS interval.

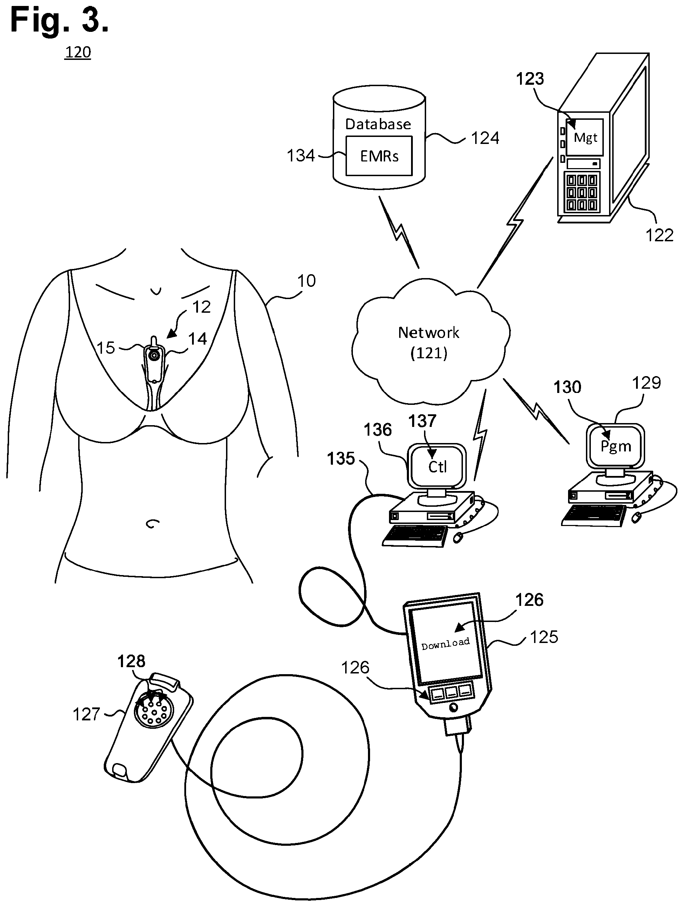

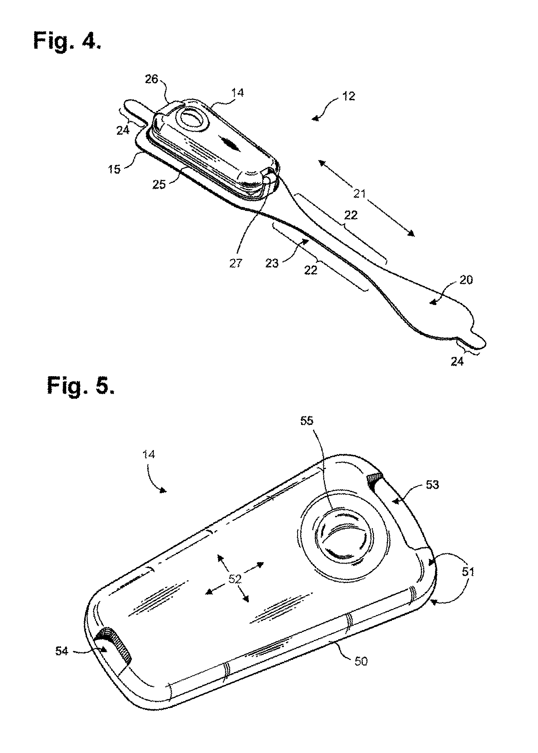

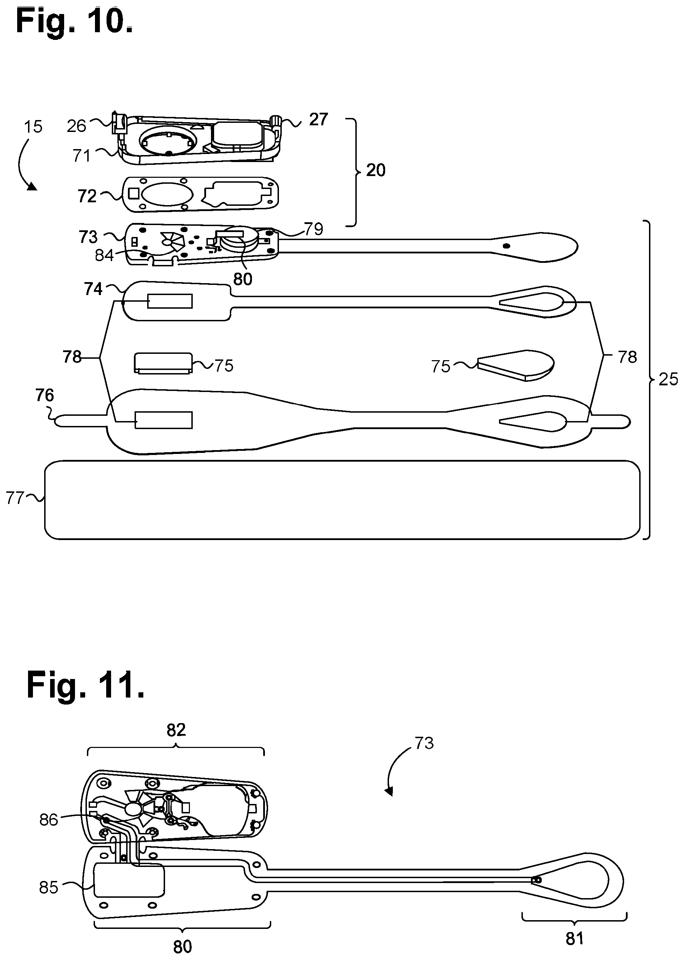

The monitor recorder 14 of the extended wear electrocardiography and physiological sensor monitor 12 senses and records the patient's ECG data into an onboard memory. Over time, disposable electrode patches 15 will require replacement and ensuring that the level of quality of ECG recording and patient service remains constant over an extended period of time is dependent upon the monitoring equipment, particularly the replacement electrode patches 15, being up to a known standard. FIGURE 3 is a functional block diagram showing a system 120 for providing a self-authenticating electrocardiography monitoring circuit in accordance with one embodiment. Self-authentication allows quality and safety expectations to be maintained. The monitor recorder 14 is a reusable component that can be fitted during patient monitoring into a non-conductive receptacle provided on the electrode patch 15, as further described *infra* with reference to FIGURE 4, and later removed for offloading of stored ECG data or to receive revised programming. The monitor recorder 14 executes an authentication protocol as part of a power up sequence, as further described *infra* with reference to FIGURE 11. Following completion of ECG monitoring, the monitor recorder 14 can be connected to a download station 125, which could be a programmer or other device that permits the retrieval of stored ECG monitoring data, execution of diagnostics on or programming of the monitor recorder 14, or performance of other functions. The monitor recorder 14 has a set of electrical contacts (not shown) that enable the monitor recorder 14 to physically interface to a set of terminals 128 on a paired receptacle 127 of the download station 125. In turn, the download station 125 executes a communications or offload program 126 ("Offload") or similar program that interacts with the monitor recorder 14 via the physical interface to retrieve the stored ECG monitoring data. The download station 125 could be a server, personal computer, tablet or handheld computer, smart mobile device, or purpose-built programmer designed specific to the task of interfacing with a monitor recorder 14. Still other forms of download station 125 are possible.

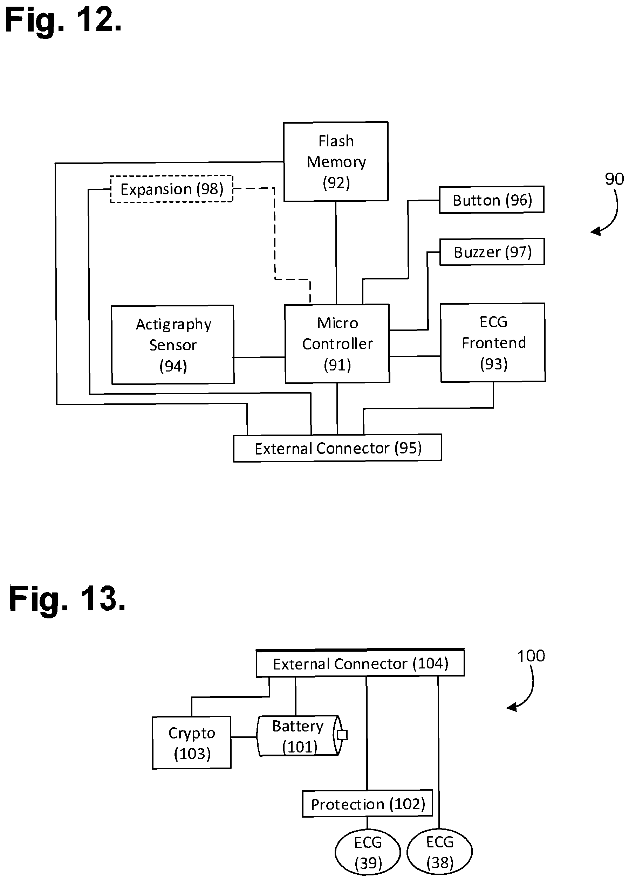

Upon retrieving stored ECG monitoring data from a monitor recorder 14, middleware first operates on the retrieved data to adjust the ECG waveform, as necessary, and to convert the retrieved data into a format suitable for use by third party post-monitoring analysis software. The formatted data can then be retrieved from the download station 125 over a hard link 135 using a control program 137 ("Ctl") or analogous application executing on a personal computer 136 or other connectable computing device, via a communications link (not shown), whether wired or wireless, or by physical transfer of storage media (not shown). The personal computer 136 or other connectable device may also execute middleware that converts ECG data and other information into a format suitable for use by a third-party post-monitoring analysis program, as further described *infra* with reference to FIGURE 13. Note that formatted data stored on the personal computer 136 would have to be maintained and safeguarded in the same manner as electronic medical records (EMRs) 134 in the secure database 124, as further discussed *infra.* In a further embodiment, the download station 125 is able to directly interface with other devices over a computer communications network 121, which could be some combination of a local area network and a wide area network, including the Internet, over a wired or wireless connection.

A client-server model could be used to employ a server 122 to remotely interface with the download station 125 over the network 121 and retrieve the formatted data or other information. The server 122 executes a patient management program 123 ("Mgt") or similar application that stores the retrieved formatted data and other information in a secure database 124 cataloged in that patient's EMRs 134. In addition, the patient management program 123 could manage a subscription service that authorizes a monitor recorder 14 to operate for a set period of time or under pre-defined operational parameters and privileges, such as described in *infra* with reference to FIGURE 13.

The patient management program 123, or other trusted application, also maintains and safeguards the secure database 124 to limit access to patient EMRs 134 to only authorized parties for appropriate medical or other uses, such as mandated by state or federal law, such as under the Health Insurance Portability and Accountability Act (HIPAA) or per the European Union's Data Protection Directive. For example, a physician may seek to review and evaluate his patient's ECG monitoring data, as securely stored in the secure database 124. The physician would execute an application program 130 ("Pgm"), such as a post-monitoring ECG analysis program, on a personal computer 129 or other connectable computing device, and, through the application 130, coordinate access to his patient's EMRs 134 with the patient management program 123. Other schemes and safeguards to protect and maintain the integrity of patient EMRs 134 are possible.

During use, the electrode patch 15 is first adhesed to the skin along the sternal midline 16 (or immediately to either side of the sternum 13). A monitor recorder 14 is then snapped into place on the electrode patch 15 to initiate ECG monitoring. FIGURE 4 is a perspective view showing an extended wear electrode patch 15 with a monitor recorder 14 in accordance with one embodiment inserted. The body of the electrode patch 15 is preferably constructed using a flexible backing 20 formed as an elongated strip 21 of wrap knit or similar stretchable material with a narrow longitudinal mid-section 23 evenly tapering inward from both sides. A pair of cut-outs 22 between the distal and proximal ends of the electrode patch 15 create a narrow longitudinal midsection 23 or "isthmus" and defines an elongated "hourglass"-like shape, when viewed from above.

The electrode patch 15 incorporates features that significantly improve wearability, performance, and patient comfort throughout an extended monitoring period. During wear, the electrode patch 15 is susceptible to pushing, pulling, and torqueing movements, including compressional and torsional forces when the patient bends forward, and tensile and torsional forces when the patient leans backwards. To counter these stress forces, the electrode patch 15 incorporates strain and crimp reliefs, such as described in commonly-assigned U.S. Patent No. 9,545,204, issued January 17, 2017, the disclosure of which is incorporated by reference. In addition, the cut-outs 22 and longitudinal midsection 23 help minimize interference with and discomfort to breast tissue, particularly in women (and gynecomastic men). The cut-outs 22 and longitudinal midsection 23 further allow better conformity of the electrode patch 15 to sternal bowing and to the narrow isthmus of flat skin that can occur along the bottom of the intermammary cleft between the breasts, especially in buxom women. The cut-outs 22 and longitudinal midsection 23 help the electrode patch 15 fit nicely between a pair of female breasts in the intermammary cleft. Still other shapes, cut-outs and conformities to the electrode patch 15 are possible.

The monitor recorder 14 removably and reusably snaps into an electrically non-conductive receptacle 25 during use. The monitor recorder 14 contains electronic circuitry for recording and storing the patient's electrocardiography as sensed via a pair of ECG electrodes provided on the electrode patch 15, such as described in commonly-assigned U.S. Patent No. 9,720,593, issued August 15, 2017, the disclosure of which is incorporated by reference. The non-conductive receptacle 25 is provided on the top surface of the flexible backing 20 with a retention catch 26 and tension clip 27 molded into the non-conductive receptacle 25 to conformably receive and securely hold the monitor recorder 14 in place.

The monitor recorder 14 includes a sealed housing that snaps into place in the non-conductive receptacle 25. FIGURE 5 is a perspective view showing the monitor recorder 14 of FIGURE 4. The sealed housing 50 of the monitor recorder 14 intentionally has a rounded isosceles trapezoidal-like shape 52, when viewed from above, such as described in commonly-assigned U.S. Design Patent No. D717955, issued November 18, 2014, the disclosure of which is incorporated by reference. The edges 51 along the top and bottom surfaces are rounded for patient comfort. The sealed housing 50 is approximately 47 mm long, 23 mm wide at the widest point, and 7 mm high, excluding a patient-operable tactile-feedback button 55. The sealed housing 50 can be molded out of polycarbonate, ABS, or an alloy of those two materials. The button 55 is waterproof and the button's top outer surface is molded silicon rubber or similar soft pliable material. A retention detent 53 and tension detent 54 are molded along the edges of the top surface of the housing 50 to respectively engage the retention catch 26 and the tension clip 27 molded into non-conductive receptacle 25. Other shapes, features, and conformities of the sealed housing 50 are possible.

The electrode patch 15 is intended to be disposable. The monitor recorder 14, however, is reusable and can be transferred to successive electrode patches 15 to ensure continuity of monitoring. The placement of the wearable monitor 12 in a location at the sternal midline 16 (or immediately to either side of the sternum 13) benefits long-term extended wear by removing the requirement that ECG electrodes be continually placed in the same spots on the skin throughout the monitoring period. Instead, the patient is free to place an electrode patch 15 anywhere within the general region of the sternum 13.

As a result, at any point during ECG monitoring, the patient's skin is able to recover from the wearing of an electrode patch 15, which increases patient comfort and satisfaction, while the monitor recorder 14 ensures ECG monitoring continuity with minimal effort. A monitor recorder 14 is merely unsnapped from a worn out electrode patch 15, the worn out electrode patch 15 is removed from the skin, a new electrode patch 15 is adhered to the skin, possibly in a new spot immediately adjacent to the earlier location, and the same monitor recorder 14 is snapped into the new electrode patch 15 to reinitiate and continue the ECG monitoring.

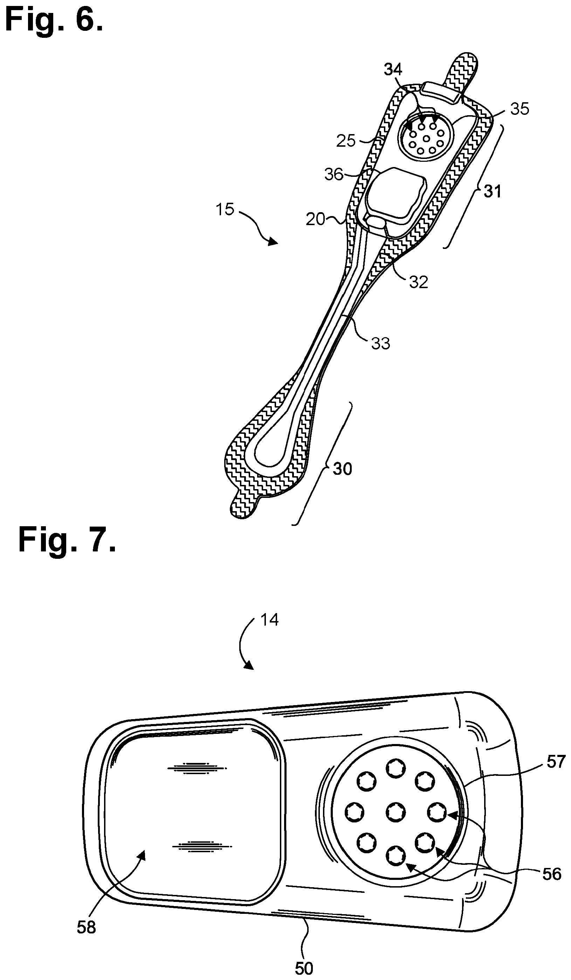

During use, the electrode patch 15 is first adhered to the skin in the sternal region. FIGURE 6 is a perspective view showing the extended wear electrode patch 15 of FIGURE 4 without a monitor recorder 14 inserted. A flexible circuit 32 is adhered to each end of the flexible backing 20. A distal circuit trace 33 and a proximal circuit trace (not shown) electrically couple ECG electrodes (not shown) to a pair of electrical pads 34. The electrical pads 34 are provided within a moisture-resistant seal 35 formed on the bottom surface of the non-conductive receptacle 25. When the monitor recorder 14 is securely received into the non-conductive receptacle 25, that is, snapped into place, the electrical pads 34 interface to electrical contacts (not shown) protruding from the bottom surface of the monitor recorder 14, and the moisture-resistant seal 35 enables the monitor recorder 14 to be worn at all times, even during bathing or other activities that could expose the monitor recorder 14 to moisture.

In addition, a battery compartment 36 is formed on the bottom surface of the non-conductive receptacle 25, and a pair of battery leads (not shown) electrically interface the battery to another pair of the electrical pads 34. The battery contained within the battery compartment 35 can be replaceable, rechargeable or disposable.

The monitor recorder 14 draws power externally from the battery provided in the non-conductive receptacle 25, thereby uniquely obviating the need for the monitor recorder 14 to carry a dedicated power source. FIGURE 7 is a bottom plan view of the monitor recorder 14 of FIGURE 4. A cavity 58 is formed on the bottom surface of the sealed housing 50 to accommodate the upward projection of the battery compartment 36 from the bottom surface of the non-conductive receptacle 25, when the monitor recorder 14 is secured in place on the non-conductive receptacle 25. A set of electrical contacts 56 protrude from the bottom surface of the sealed housing 50 and are arranged in alignment with the electrical pads 34 provided on the bottom surface of the non-conductive receptacle 25 to establish electrical connections between the electrode patch 15 and the monitor recorder 14. In addition, a seal coupling 57 circumferentially surrounds the set of electrical contacts 56 and securely mates with the moisture-resistant seal 35 formed on the bottom surface of the non-conductive receptacle 25.

The placement of the flexible backing 20 on the sternal midline 16 (or immediately to either side of the sternum 13) also helps to minimize the side-to-side movement of the wearable monitor 12 in the left- and right-handed directions during wear. To counter the dislodgment of the flexible backing 20 due to compressional and torsional forces, a layer of non-irritating adhesive, such as hydrocolloid, is provided at least partially on the underside, or contact, surface of the flexible backing 20, but only on the distal end 30 and the proximal end 31. As a result, the underside, or contact surface of the longitudinal midsection 23 does not have an adhesive layer and remains free to move relative to the skin. Thus, the longitudinal midsection 23 forms a crimp relief that respectively facilitates compression and twisting of the flexible backing 20 in response to compressional and torsional forces. Other forms of flexible backing crimp reliefs are possible.

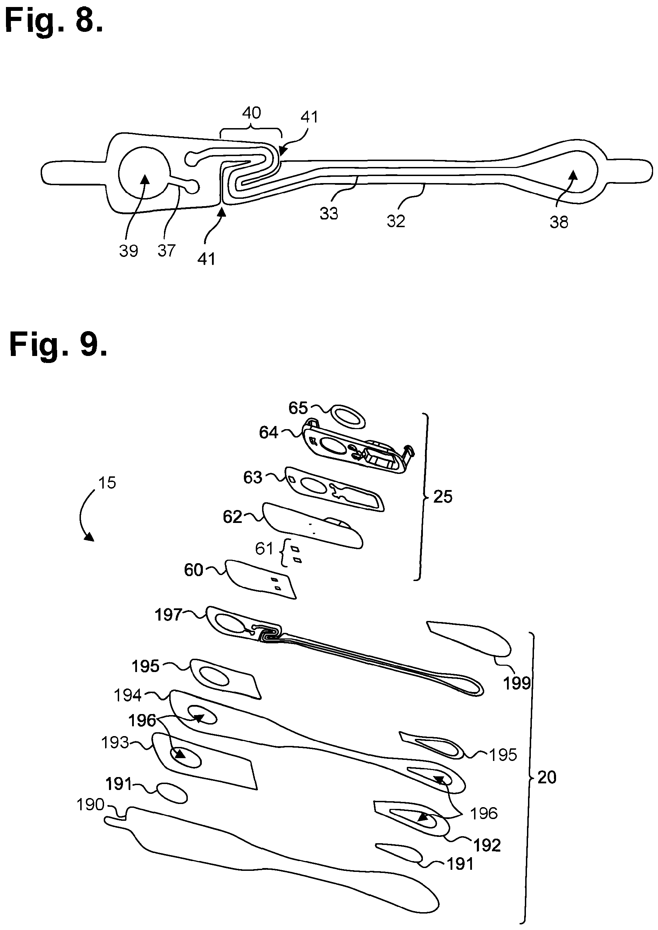

Unlike the flexible backing 20, the flexible circuit 32 is only able to bend and cannot stretch in a planar direction. The flexible circuit 32 can be provided either above or below the flexible backing 20. FIGURE 8 is a top view showing the flexible circuit 32 of the extended wear electrode patch 15 of FIGURE 4 when mounted above the flexible backing 20. A distal ECG electrode 38 and proximal ECG electrode 39 are respectively coupled to the distal and proximal ends of the flexible circuit 32. A strain relief 40 is defined in the flexible circuit 32 at a location that is partially underneath the battery compartment 36 when the flexible circuit 32 is affixed to the flexible backing 20. The strain relief 40 is laterally extendable to counter dislodgment of the ECG electrodes 38, 39 due to tensile and torsional forces. A pair of strain relief cutouts 41 partially extend transversely from each opposite side of the flexible circuit 32 and continue longitudinally towards each other to define in 'S'-shaped pattern, when viewed from above. The strain relief respectively facilitates longitudinal extension and twisting of the flexible circuit 32 in response to tensile and torsional forces. Other forms of circuit board strain relief are possible.

When provided above the flexible backing 20, adhesive layers are provided above and below the flexible circuit 32. FIGURE 9 is an exploded view showing the component layers of the electrode patch 15 of FIGURE 4. The flexible backing 20 is constructed of a wearable gauze, latex, or similar wrap knit or stretchable and wear-safe material 54, such as a Tricot-type linen with a pressure sensitive adhesive (PSA) on the underside, or contact, surface. The wearable material 194 is coated with a layer 193, 192 of non-irritating adhesive, such as hydrocolloid, to facilitate long-term wear. The hydrocolloid, for instance, is typically made of mineral oil, cellulose and water and lacks any chemical solvents, so should cause little itching or irritation. Moreover, hydrocolloid is thicker and more gel-like than most forms of PSA and provides cushioning between the relatively rigid and unyielding non-conductive receptacle 25 and the patient's skin. In a further embodiment, the layer of non-irritating adhesive can be contoured, such as by forming the adhesive with a concave or convex cross-section; surfaced, such as through stripes or crosshatches of adhesive, or by forming dimples in the adhesive's surface; or applied discontinuously, such as with a formation of discrete dots of adhesive.

As described *supra* with reference to FIGURE 8, a flexible circuit can be adhered to either the outward facing surface or the underside, or contact, surface of the flexible backing 20. For convenience, a flexible circuit 197 is shown, such as in FIGURE 9, relative to the outward facing surface of the wearable material 194 and is adhered respectively on a distal end by a distal electrode seal 195 and on a proximal end by a proximal electrode seal 195. In a further embodiment, the flexible circuit 197 can be provided on the underside, or contact, surface of the wearable material 194. Through the electrode seals, only the distal and proximal ends of the flexible circuit 197 are attached to the wearable material 194, which enables the strain relief 50 (shown in FIGURE 8) to respectively longitudinally extend and twist in response to tensile and torsional forces during wear. Similarly, the layer 193, 192 of non-irritating adhesive is provided on the underside, or contact, surface of the wearable material 194 only on the proximal and distal ends, which enables the longitudinal midsection 23 (shown in FIGURE 4) to respectively bow outward and away from the sternum 13 or twist in response to compressional and torsional forces during wear. In a further embodiment, the layer 193, 192 of non-irritating adhesive is provided along a length of the wearable material 194.

A pair of openings 196 is defined on the distal and proximal ends of the wearable material 194 and layer 193, 192 of non-irritating adhesive for ECG electrodes 38, 39 (shown in FIGURE 8). The openings 196 serve as "gel" wells with a layer of hydrogel 191 being used to fill the bottom of each opening 196 as a conductive material that aids electrode signal pick up. The entire underside, or contact, surface of the flexible backing 20 is protected prior to use by a liner layer 190 that is peeled away.

The non-conductive receptacle 25 includes a main body 64 that is molded out of polycarbonate, ABS, or an alloy of those two materials to provide a high surface energy to facilitate adhesion of an adhesive seal 63. The main body 64 is attached to a battery printed circuit board 62 by the adhesive seal 63 and, in turn, the battery printed circuit board 62 is adhesed to the flexible circuit 197 with an upper flexible circuit seal 60. A pair of conductive transfer adhesive points 61 or, alternatively, metallic rivets or similar conductive and structurally unifying components, connect the circuit traces 33, 37 (shown in FIGURE 8) of the flexible circuit 197 to the battery printed circuit board 62. The main body 64 has a retention catch 26 and tension clip 27 (shown in FIGURE 4) that fixably and securely receive a monitor recorder 14 (shown in FIGURE 4), and includes a recess within which to circumferentially receive a die cut gasket 65, either rubber, urethane foam, or similar suitable material, to provide a moisture resistant seal to the set of pads 34 (shown in FIGURE 6).

Together, the components of the electrode patch 15, as described above, form a signal path for transmission of ECG data sensed by the electrodes, to the recorder monitor for collection and transfer to a download station. Reducing a number of components in the signal path can simplify fabrication and decrease manufacturing costs, as well as enhance noise reduction FIGURE 10 is an alternative exploded view showing the component layers of the electrode patch 15 of FIGURE 4. The non-conductive receptacle 20 is provided on a top surface of the flexible backing 25 to conformably receive and securely hold the monitor recorder 14 (shown in FIGURE 4) in place. The flexible backing 25 can include an integrated flex circuit 73, electrode seal 74, patient adhesive 76, and a liner layer 77. The flexible backing can also include ECG electrodes (not shown) and hydrogel 75, which is in contact with the ECG electrodes.

The integrated flex circuit 73 can be constructed from material, such as polyester substrate and silver ink, and can include a pair of circuit traces (not shown), one on a distal end and one on a proximal end of the integrated flex circuit. The circuit traces electrically couple the ECG electrodes to a pair of electrical pads 84 on the integrated flex circuit. Specifically, the silver ink is applied to the polyester substrate of the integrated flex circuit 73 for electrical conductivity of the circuit traces (shown in FIGURE 8) using screen-printing, pad-printing, or flexography. Other types of material, ink and means for electrical conductivity are possible. Two or more of the electrical pads are formed on the outward facing surface of the integrated flex circuit, which can also form a bottom surface of the non-conductive receptacle 20. Additionally, one or more electrical pads can also be located on a contact surface of the integrated flexible circuit.

A battery 79 is adhered directly to the outward facing surface of the integrated flex circuit 73, removing the need for a battery printed circuit board, adhesive points and a flexible circuit seal, as shown in FIGURE 9, which is described above in detail. The battery 79 can be soldered to the integrated flex circuit 73 or alternatively, the battery 79 is affixed to the integrated flex circuit 73 via a clip 80. Other means for attaching the battery 79 to the integrated flex circuit 73 are possible.

A layer of patient adhesive 76 is provided on the contact surface of the integrated flex circuit 73 via one or more electrode seals 74. The electrode seal 74 includes a double sided layer of adhesive to connect the integrated flex circuit 73 and the adhesive layer 76. The adhesive layer 76 is a type of wearable material coated on a bottom, or contact, surface with a layer of non-irritating adhesive, such as hydrocolloid. The wearable material can include gauze, latex, wrap knit, or other types of stretchable and wear-safe material, such as a Tricot-type linen with a pressure sensitive adhesive on the underside, or contact surface The electrode seal 74 and adhesive layer 76 can each cover the entire contact surface of the integrated flex circuit 73 or merely a portion, such as on proximal and distal ends of the integrated flex circuit 73.

Further, openings 78 are defined on the distal and proximal ends of each of the electrode seal 74 and adhesive layer 76 for ECG electrodes 38, 39 (shown in FIGURE 8). The openings serve as "gel" wells with a layer of hydrogel 75 used to fill the bottom of each opening as a conductive material that aids signal pick up by the electrodes, which are electrically coupled to the circuit traces. The entire underside, or contact, surface of the flexible backing 20 is protected prior to use by a liner layer 77 that is peeled away.

The non-conductive receptacle 20 includes a main body 71 that is molded out of polycarbonate, ABS, or an alloy of those two materials to provide a high surface energy to facilitate adhesion of an adhesive seal 72. The main body 71 is adhesed to the integrated flex circuit 73 via the adhesive seal 72 and has a retention catch 26 and tension clip 27 (shown in FIGURE 4) that fixably and securely receive a monitor recorder 14 (not shown). The main body 71 also includes a recess within which to circumferentially receive a die cut gasket (not shown), either rubber, urethane foam, or similar suitable material, to provide a moisture resistant seal to the set of pads 34 (shown in FIGURE 6).

Decreasing a number of components in the electrode patch can help decrease noise in the collection of ECG data from a patient, which is extremely important because some types of noise can look like certain kinds of arrythmias. The integrated flex circuit includes a battery, removing the need for a separate printed circuit board. FIGURE 11 is an exploded view showing the integrated flex circuit 73 of the electrode patch of FIGURE 10 The integrated flex circuit 73 is formed from a single piece of material, such as polyester substrate. As described above with respect to FIGURE 4, a pair of cut-outs between distal and proximal ends of the electrode patch create a narrow longitudinal midsection and defines an elongated "hourglass"-like shape of the integrated flex circuit, in one embodiment. The integrated flex circuit 73 can also be shaped in the elongated "hourglass" -like shape, consistent with the electrode patch. Further, an upper portion 80 of the "hourglass" shaped integrated flex circuit is formed on proximal end and sized to hold the electrically non-conductive receptacle (not shown). The upper 80 portion has a longer and wider profile than the lower part 81 of the "hourglass," which is sized primarily to allow just the placement of an ECG electrode. Additionally, the integrated flex circuit 73 includes a duplicate 82 of the upper portion 80 that is a mirror image and connected to the upper portion along one side. The mirror copy 82 of the upper portion is folded over the upper portion 80. Other shapes of the electrode patch and the integrated flex circuit are possible, including rectangular, square, oval, and circular.

Each of the upper portion and the mirror copy of the upper portion can include electrical pads 85, 86, which establish electrical connections between the electrode patch and the monitor recorder. Specifically, the electrical pads on the mirror copy of the upper portion interface to electrical contacts (not shown) protruding from the bottom surface of the monitor recorder.

In a further embodiment, one or both of the electrode patch 15 and integrated flex circuit 73 form a different shape, such as a long rectangular strip or another shape. In such configuration, a portion of the integrated flex circuit 73 is designated as the upper portion and duplicated to form a mirror image, which is folded over the designed upper portion.

Due to the flexible nature of the integrated circuit, a stiffener is used to prevent unnecessary bending of the circuit, such as when a patient presses a tactile feedback button on the monitor to mark events or to perform other functions. In one embodiment, the stiffener can be the same shape and size as the integrated flex circuit and can be made from epoxy laminate sheets or fiberglass. In another embodiment, the upper portion is folded over a stiffener, which is located between the upper portion and the mirror copy, and laminated together.

When inserted in the non-conductive receptacle of the electrode patch, the monitor recorder performs ECG monitoring and other functions through a micro controlled architecture. FIGURE 12 is a functional block diagram showing the component architecture of the circuitry 90 of the monitor recorder 14 of FIGURE 4. The circuitry 90 is externally powered through a battery provided in the non-conductive receptacle 25 (shown in FIGURE 6). Both power and raw ECG signals, which originate in the pair of ECG electrodes 38, 39 (shown in FIGURE 8) on the distal and proximal ends of the electrode patch 15, are received through an external connector 95 that mates with a corresponding physical connector on the electrode patch 15. The external connector 95 includes the set of electrical contacts 56 (shown in FIGURE 7) that protrude from the bottom surface of the sealed housing 50 and which physically and electrically interface with the set of pads 34 (shown in FIGURE 6) provided on the bottom surface of the non-conductive receptacle 25. The external connector 95 includes electrical contacts 56 for data download, microcontroller communications, power, analog inputs, and a peripheral expansion port. The arrangement of the pins on the external connector 95 of the monitor recorder 14 and the device into which the monitor recorder 14 is attached, whether an electrode patch 15 or download station 125 (shown in FIGURE 3), follow the same electrical pin assignment convention to facilitate interoperability. The external connector 65 also serves as a physical interface to a download station that permits the retrieval of stored ECG monitoring data, communication with the monitor recorder 14, and performance of other functions.

Operation of the circuitry 90 of the monitor recorder 14 is managed by a microcontroller 91. The micro-controller 91 includes a program memory unit containing internal flash memory that is readable and writeable. The internal flash memory can also be programmed externally. The micro-controller 91 draws power externally from the battery provided on the electrode patch 15 via a pair of the electrical contacts 56 The microcontroller 91 connects to the ECG front end circuit 93 that measures raw cutaneous electrical signals and generates an analog ECG signal representative of the electrical activity of the patient's heart over time.

The circuitry 90 of the monitor recorder 14 also includes a flash memory 92, which the micro-controller 91 uses for storing ECG monitoring data and other physiology and information. The flash memory 92 also draws power externally from the battery provided on the electrode patch 15 via a pair of the electrical contacts 56. Data is stored in a serial flash memory circuit, which supports read, erase and program operations over a communications bus. The flash memory 92 enables the microcontroller 91 to store digitized ECG data. The communications bus further enables the flash memory 92 to be directly accessed externally over the external connector 95 when the monitor recorder 14 is interfaced to a download station.

The circuitry 90 of the monitor recorder 14 further includes an actigraphy sensor 94 implemented as a 3-axis accelerometer. The accelerometer may be configured to generate interrupt signals to the microcontroller 91 by independent initial wake up and free fall events, as well as by device position. In addition, the actigraphy provided by the accelerometer can be used during post-monitoring analysis to correct the orientation of the monitor recorder 14 if, for instance, the monitor recorder 14 has been inadvertently installed upside down, that is, with the monitor recorder 14 oriented on the electrode patch 15 towards the patient's feet, as well as for other event occurrence analyses, such as described in commonly-assigned U.S. Patent No. 9,737,224, issued August 22, 2017, the disclosure of which is incorporated by reference.

The microcontroller 91 includes an expansion port 98 that also utilizes the communications bus. External devices, separately drawing power externally from the battery provided on the electrode patch 15 or other source, can interface to the microcontroller 91 over the expansion port in half duplex mode. For instance, an external physiology sensor can be provided as part of the circuitry 90 of the monitor recorder 14, or can be provided on the electrode patch 15 with communication with the micro-controller 91 provided over one of the electrical contacts 56. The physiology sensor can include a SpO₂ sensor, blood pressure sensor, temperature sensor, respiratory rate sensor, glucose sensor, airflow sensor, volumetric pressure sensing, or other types of sensor or telemetric input sources. For instance, the integration of an airflow sensor is described in commonly-assigned U.S. Patent No. 9,364,155, issued June 14, 2016, the disclosure which is incorporated by reference In a further embodiment, a wireless interface for interfacing with other wearable (or implantable) physiology monitors, as well as data offload and programming, can be provided as part of the circuitry 90 of the monitor recorder 14, or can be provided on the electrode patch 15 with communication with the micro-controller 91 provided over one of the electrical contacts 56, such as described in commonly-assigned U.S. Patent No. 9,433,367, issued September 6, 2016 the disclosure of which is incorporated by reference.

Finally, the circuitry 90 of the monitor recorder 14 includes patient-interfaceable components, including a tactile feedback button 96, which a patient can press to mark events or to perform other functions, and a buzzer 67, such as a speaker, magnetic resonator or piezoelectric buzzer. The buzzer 97 can be used by the microcontroller 91 to output feedback to a patient such as to confirm power up and initiation of ECG monitoring. Still other components as part of the circuitry 90 of the monitor recorder 14 are possible.

While the monitor recorder 14 operates under micro control, some of the electrical components of the electrode patch 15 operate passively, while others are active. FIGURE 13 is a functional block diagram showing the circuitry 100 of the extended wear electrode patch 15 of FIGURE 4. The circuitry 100 of the electrode patch 15 is electrically coupled with the circuitry 90 of the monitor recorder 14 through an external connector 104. The external connector 104 is terminated through the set of pads 34 provided on the bottom of the non-conductive receptacle 25, which electrically mate to corresponding electrical contacts 56 protruding from the bottom surface of the sealed housing 50 to electrically interface the monitor recorder 14 to the electrode patch 15.

The circuitry 100 of the electrode patch 15 performs three primary functions. First, a battery 101 is provided in a battery compartment formed on the bottom surface of the non-conductive receptacle 25. The battery 101 is electrically interfaced to the circuitry 90 of the monitor recorder 14 as a source of external power. The unique provisioning of the battery 101 on the electrode patch 15 provides several advantages. First, the locating of the battery 101 physically on the electrode patch 15 lowers the center of gravity of the overall wearable monitor 12 and thereby helps to minimize shear forces and the effects of movements of the patient and clothing. Moreover, the housing 50 of the monitor recorder 14 is sealed against moisture and providing power externally avoids having to either periodically open the housing 50 for the battery replacement, which also creates the potential for moisture intrusion and human error, or to recharge the battery, which can potentially take the monitor recorder 14 off line for hours at a time. In addition, the electrode patch 15 is intended to be disposable, while the monitor recorder 14 is a reusable component. Each time that the electrode patch 15 is replaced, a fresh battery is provided for the use of the monitor recorder 14, which enhances ECG monitoring performance quality and duration of use. Finally, the architecture of the monitor recorder 14 is open, in that other physiology sensors or components can be added by virtue of the expansion port of the microcontroller 91. Requiring those additional sensors or components to draw power from a source external to the monitor recorder 14 keeps power considerations independent of the monitor recorder 14. Thus, a battery of higher capacity could be introduced when needed to support the additional sensors or components without effecting the monitor recorders circuitry 90.

Second, the pair of ECG electrodes 38, 39 respectively provided on the distal and proximal ends of the flexible circuit 32 are electrically coupled to the set of pads 34 provided on the bottom of the non-conductive receptacle 25 by way of their respective circuit traces 33, 37. The signal ECG electrode 39 includes a protection circuit 102, which is an inline resistor that protects the patient from excessive leakage current.

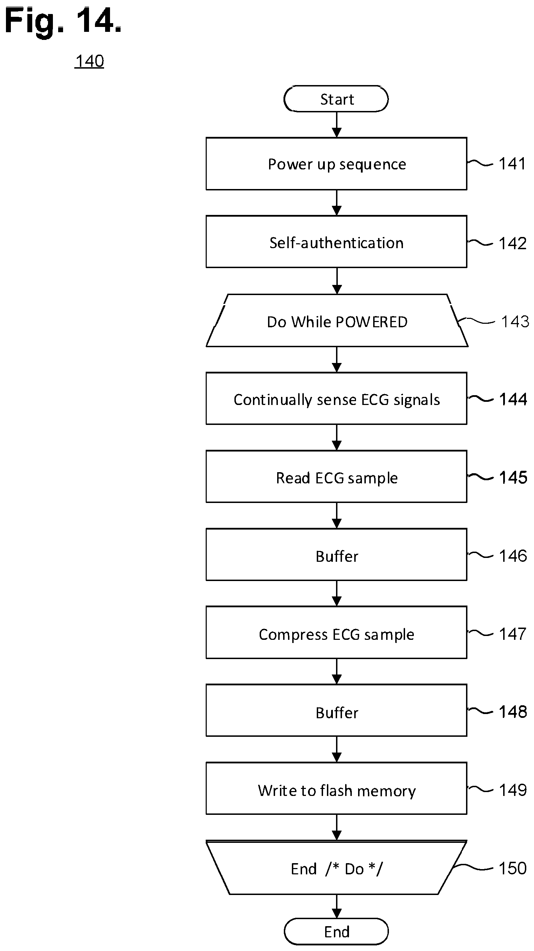

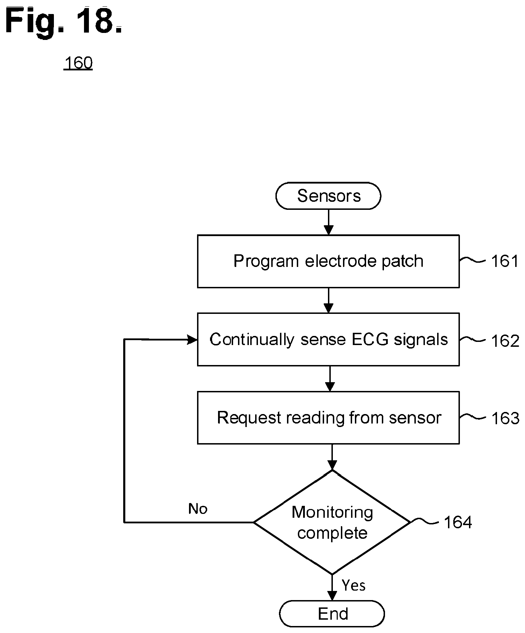

Last, in a further embodiment, the circuitry 100 of the electrode patch 15 includes a cryptographic circuit 103 to authenticate an electrode patch 15 for use with a monitor recorder 14. The cryptographic circuit 103 includes a device capable of secure authentication and validation. The cryptographic device 103 ensures that only genuine, non-expired, safe, and authenticated electrode patches 15 are permitted to provide monitoring data to a monitor recorder 14. Further, the cryptographic circuit can include readable and writeable memory for storing data received from the monitor recorder or external sensors, or providing instructions to the monitor recorder and external sensors, as further described below in detail below with reference to FIGURES 14 and 18. However, the readable and writeable memory for storing data can be provided separately from the cryptographic circuit.

The monitor recorder 14 continuously monitors the patient's heart rate and physiology. FIGURE 14 is a flow diagram showing a monitor recorder-implemented method 140 for monitoring ECG data for use in the monitor recorder 14 of FIGURE 4. Initially, upon being connected to the set of pads 34 provided with the non-conductive receptacle 25 when the monitor recorder 14 is snapped into place, the microcontroller 91 executes a power up sequence (step 141) and performs self-authentication of the electrode patch 15 (step 142). During the power up sequence, the voltage of the battery 101 is checked, the state of the flash memory 92 is confirmed, both in terms of operability check and available capacity, and microcontroller operation is diagnostically confirmed.

Self-authentication is performed between the microcontroller 91 and the electrode patch 15 each time that the monitor recorder 14 is inserted into an electrode patch 15 (or other accessory) to ensure patient safety. An authenticated patch will conform to product quality standards, as well as applicable federal regulatory quality requirements and international standards, such as ISO 13485, IEC 60601-2-47 and IEC 60601-1. Quality assurance, through self-authentication, is crucial, as electrode patches 15 and other accessories may be authorized, but may not necessarily be manufactured, by the entity ultimately responsible for quality standards compliance. Self-authentication mitigates the risk of incorrect device output due to non-compliant accessories.

In one embodiment, the micro-controller91 contains a private key or a precomputed digest, of which the electrode patch 15 (or other accessory) will have a copy. To authenticate an electrode patch 15 (or other accessory), the micro-controller 91 will challenge the electrode patch 15 (or other accessory) using a code hashed with the private key or precomputed digest. If the electrode patch 15 (or other accessory) responds correctly, the micro-controller 91 will continue with normal program execution. Otherwise, the monitor recorder 14 will signal an error condition, such as chirping the buzzer 97 to notify the patient. Failing self-authentication, other actions could also be taken.

In a further embodiment, an electrode patch 15 (or other accessory, such as a separate physiological sensor) can be set to operate for only a certain period of time. Upon authentication, the monitor recorder 14 will run with that electrode patch 15 (or other accessory) until the electrode patch 15 (or other accessory) is depleted. If the electrode patch 15 (or other accessory) is detected to be expired during self-authentication, the monitor recorder 14 will fail to operate or signal an error condition. To cause an electrode patch 15 (or other accessory) to expire after a certain amount of time has elapsed, the micro-controller 91 periodically writes into the read-only memory (ROM) of the cryptographic circuit 103. After the data in the ROM written by the micro-controller 91 has reached a certain fullness, the micro-controller 91 will turn off to ensure that an expired electrode patch 15 (or other accessory) does not create an unsafe condition, such as an incorrect output. Still other forms of authentication and device expiration are possible.

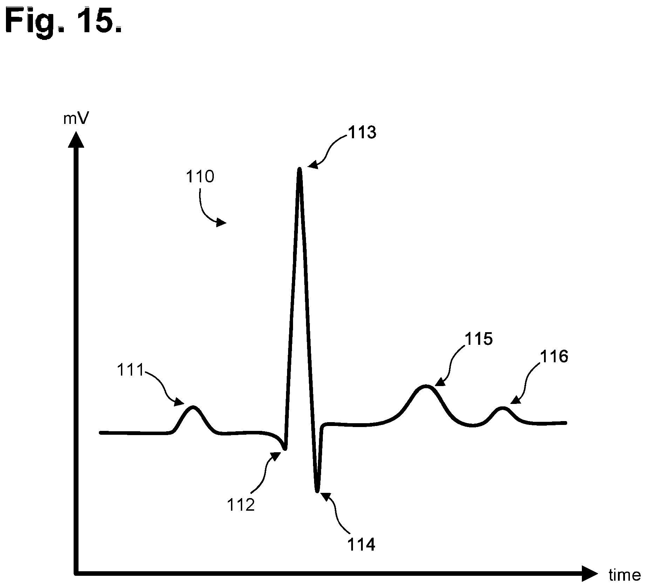

Following satisfactory completion of the power up sequence, an iterative processing loop (steps 143-150) is continually executed by the microcontroller 91. During each iteration (step 143) of the processing loop, the ECG frontend 93 (shown in FIGURE 9) continually senses the cutaneous ECG electrical signals (step 144) via the ECG electrodes 38, 39 and is optimized to maintain the integrity of the P-wave. A sample of the ECG signal is read (step 145) by the microcontroller 91 by sampling the analog ECG signal output front end 93. FIGURE 15 is a graph showing, by way of example, a typical ECG waveform 110. The x-axis represents time in approximate units of tenths of a second. The *y*-axis represents cutaneous electrical signal strength in approximate units of millivolts. The P-wave 111 has a smooth, normally upward, that is, positive, waveform that indicates atrial depolarization. The QRS complex usually begins with the downward deflection of a Q wave 112, followed by a larger upward deflection of an R-wave 113, and terminated with a downward waveform of the S wave 114, collectively representative of ventricular depolarization. The T wave 115 is normally a modest upward waveform, representative of ventricular depolarization, while the U wave 116, often not directly observable, indicates the recovery period of the Purkinje conduction fibers.

Sampling of the R-to-R interval enables heart rate information derivation. For instance, the R-to-R interval represents the ventricular rate and rhythm, while the P-to-P interval represents the atrial rate and rhythm. Importantly, the PR interval is indicative of atrioventricular (AV) conduction time and abnormalities in the PR interval can reveal underlying heart disorders, thus representing another reason why the P-wave quality achievable by the extended wear ambulatory electrocardiography and physiological sensor monitor described herein is medically unique and important. The long-term observation of these ECG indicia, as provided through extended wear of the wearable monitor 12, provides valuable insights to the patient's cardiac function and overall well-being.

Returning to the discussion with respect to FIGURE 14, each sampled ECG signal, in quantized and digitized form, is temporarily staged in buffer (step 146), pending compression preparatory to storage in the flash memory 92 (step 147). Following compression, the compressed ECG digitized sample is again buffered (step 148), then written to the flash memory 92 (step 149) using the communications bus. Processing continues (step 150), so long as the monitoring recorder 14 remains connected to the electrode patch 15 (and storage space remains available in the flash memory 92), after which the processing loop is exited and execution terminates. Still other operations and steps are possible.

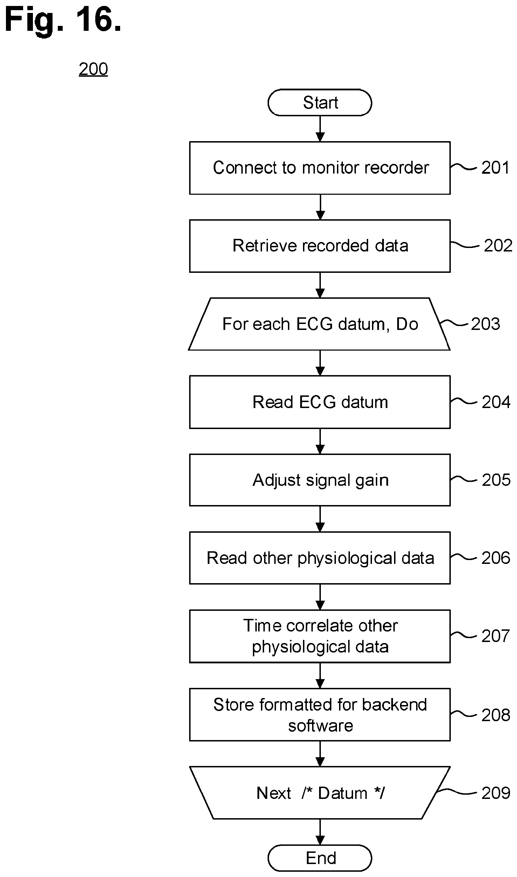

Once recorded, the ECG data can be offloaded and processed. FIGURE 16 is a flow diagram showing a method 200 for offloading and converting ECG and other physiological data from an extended wear electrocardiography and physiological sensor monitor in accordance with one embodiment. The method 150 can be implemented in software and execution of the software can be performed on a download station 125, which could be a programmer or other device, or a computer system, including a server 122 or personal computer 129, such as further described *supra* with reference to FIGURE 3, as a series of process or method modules or steps. For convenience, the method 200 will be described in the context of being performed by a personal computer 136 or other connectable computing device (shown in FIGURE 3) as middleware that converts ECG data and other information into a format suitable for use by a third-party post-monitoring analysis program. Execution of the method 200 by a computer system would be analogous *mutatis mutandis.*

Initially, the download station 125 is connected to the monitor recorder 14 (step 201), such as by physically interfacing to a set of terminals 128 on a paired receptacle 127 or by wireless connection, if available. The data stored on the monitor recorder 14, including ECG and physiological monitoring data, other recorded data, and other information are retrieved (step 202) over a hard link 135 using a control program 137 ("Ctl") or analogous application executing on a personal computer 136 or other connectable computing device.

The data retrieved from the monitor recorder 14 is in a proprietary storage format and each datum of recorded ECG monitoring data, as well as any other physiological data or other information, must be converted, so that the data can be used by a third-party post-monitoring analysis program. Each datum of ECG monitoring data is converted by the middleware (steps 203-209) in an iterative processing loop. During each iteration (step 203), the ECG datum is read (step 204) and, if necessary, the gain of the ECG signal is adjusted (step 205) to compensate, for instance, for relocation or replacement of the electrode patch 15 during the monitoring period.

In addition, depending upon the configuration of the wearable monitor 12, other physiological data (or other information), including patient events, such as a fall, peak activity level, detection of patient activity levels and states, sedentary detection and so on, may be recorded along with the ECG monitoring data. For instance, actigraphy data may have been sampled by the actigraphy sensor 94 based on a sensed event occurrence, such as a sudden change in orientation due to the patient taking a fall. In response, the monitor recorder 14 will embed the actigraphy data samples into the stream of data, including ECG monitoring data that is recorded to the flash memory 92 by the micro-controller 91. Post-monitoring, the actigraphy data is temporally matched to the ECG data to provide the proper physiological context to the sensed event occurrence. As a result, the three-axis actigraphy signal is turned into an actionable event occurrence that is provided, through conversion by the middleware, to third party post-monitoring analysis programs, along with the ECG recordings contemporaneous to the event occurrence. Other types of processing of the other physiological data (or other information) are possible.

Thus, during execution of the middleware, any other physiological data (or other information) that has been embedded into the recorded ECG monitoring data is read (step 206) and time-correlated to the time frame of the ECG signal s that occurred at the time that the other physiological data (or other information) was noted (step 207). Finally, the ECG datum, signal gain adjusted, if appropriate, and other physiological data, if applicable and as time-correlated, are stored in a format suitable to the backend software (step 208) used in post-monitoring analysis. Processing continues (step 209) for each remaining ECG datum, after which the processing loop is exited and execution terminates.

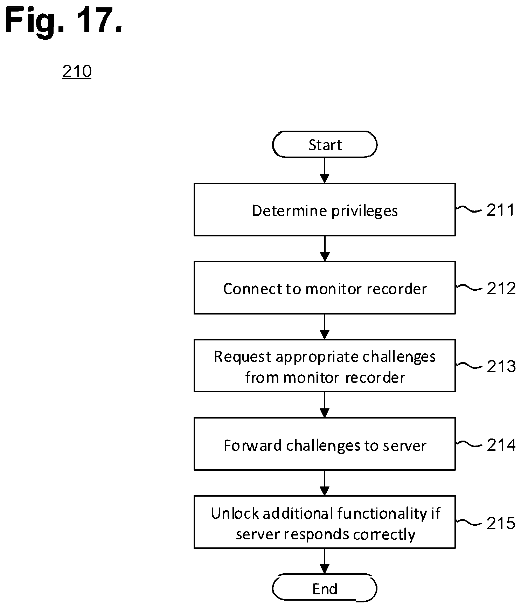

Additionally, in one embodiment, the patient management program 123 could manage a subscription service that authorizes a monitor recorder 14 to operate for a set period of time or under pre-defined operational parameters and privileges. FIGURE 17 is a flow diagram showing a method 210 for providing a self-authenticating electrocardiography monitoring circuit in accordance with one embodiment. The method 210 can be implemented in software and execution of the software can be performed on a download station 125, which could be a programmer or other device, or a computer system, including a server 122 or personal computer 129, such as further described *supra* with reference to FIGURE 3, as a series of process or method modules or steps. For convenience, the method 210 will be described in the context of being performed by a download station 125. Execution of the method 210 by a computer system would be analogous *mutatis mutandis.*

In terms of a subscription business model, devices, such as the monitor recorder 14, could be sold at low cost, and a patient would only need to pay a monitoring service provider when they want to use their device. The subscription service could be implemented by the server 122 or other comparable computer system, which would provide a user interface to patients through which they could select and purchase desired additional functionality. A patient is able to "reload" their device with more time or pre-defined operational parameters and privileges using a challenge response authentication scheme through a download station 125. Initially, the patient determines what pre-defined operational parameters or privileges he wishes to purchase (step 211), such as running time, number of uses, monitoring features, and so on, and the patient's selections are provided to the server 122 using, for instance, a personal computer 129, or similar device. The download station 125 connects to the monitor recorder 14 (step 212), such as by physically interfacing to a set of terminals 128 on a paired receptacle 127 or by wireless connection, if available. The download station 125 communicate with the monitor recorder 14 and requests appropriate challenges (step 213), which are forwarded to the patient management program 123 executing on the server 122. Upon receiving (step 214) the forwarded challenges, the monitor recorder 14 will only unlock the requested additional functionality if the server 122 responds correctly (step 215). Thus, the server 122 can preclude authorizing the additional functionality by providing an incorrect response. Other types of pre-defined operational parameter and privilege authorization schemes are possible.

In a further embodiment, physiological data can be collected via one or more physiological sensors, such as a SpO₂ sensor, blood pressure sensor, temperature sensor, respiratory rate sensor, glucose sensor, airflow sensor, volumetric pressure sensing, or other types of sensor or telemetric input sources continually or on demand as instructed by the electrode patch. Generally, continually obtaining physiological data from some types of sensors, such as a high resolution temperature sensor is not optimal nor preferred due to the large amounts of processing and memory required to obtain and store the data. However, sampling of the data randomly or at predetermined times as instructed by the electrode patch makes obtaining such data possible using the extended wear device, such as the electrocardiography and physiological sensor monitor. FIGURE 18 is a flow diagram showing, by way of example, a process 160 for obtaining physiological data from a physiological sensor. Memory provided on the electrode patch is programmed (step 161) to enable and disable features, including obtaining physiological measurements via at least one physiological sensor at different times over a period of time. In one embodiment, the authentication or crypto circuit 103 (shown in FIGURE 13) on the electrode patch includes programmable read and write memory space. However, in a different embodiment, the programmable read and write memory is provided separate from the crypto circuit. In one example, the programmed features can include predetermined times or a request for random times at which data is to be obtained via a physiological monitor. Other programmable features are also possible.

Once the monitor is applied to a patient and the electrode patch is authenticated by the monitor recorder, the electrocardiography and physiological sensor monitor continually senses (step 162) ECG signals upon authentication until the electrode patch is determined to be expired or is otherwise terminated. Meanwhile, the electrode patch can request (step 163) readings of physiological data from one or more physiological sensors provided as part of the circuitry of the monitor recorder or provided on the electrode patch. Alternatively or in addition, the physiological data can be wirelessly connected or external to the electrocardiography and physiological sensor monitor. When provided on the electrode patch, the physiological sensor can communicate with the micro-controller of the monitor recorder via one or more of the electrical contacts to obtain and store the received physiological data.

The request for data from the electrode patch can be provided based on a set of sampling instructions preprogrammed on the electrode patch, including instructions to request the physiological data randomly or at predetermined times, as described above. Other types and kinds of instructions are possible for sampling, such as requesting a reading from one or more of the physiological sensors when the patient presses the patient tactile feedback button (shown in FIGURE 4) to mark events, such as a cardiac episode, when a user is out of breath or feels his heart racing. The electrode patch can be preprogrammed at the time of manufacture, or after manufacture and prior to distribution to a patient. Based on the data request, the physiological sensor for which the request was directed, obtains a reading of the corresponding physiological data, which is then provided to the monitor recorder for storage and later downloading via a download station with the ECG data.

During monitoring of the ECG signals and physiological data, a determination is made as to whether all monitoring is complete (step 164). For example, monitoring can be complete when the electrode patch expires, such as based on an amount of data obtained or recorded, a new electrode patch should replace the current electrode patch, or a predetermined time for monitoring has been reached, such as described in commonly-assigned U.S. Patent Application Publicatin No. 20190099105, filed December 3, 2018, pending, the disclosure of which is incorporated by reference. If monitoring is complete, no further ECG signals or physiological data are recorded. However, if not complete, the ECG signals are continually recorded (step 162), while the physiological data is obtained (step 163) according to instructions from the electrode patch.

In one embodiment, different types of physiological sensors can be incorporated into the electrode patch 15 for use with the monitor recorder 14 as custom electrode patches. Specifically, each electrode patch can include a different physiological sensor and the electrode patches can be interchanged to work with the monitor recorder to obtain different types of physiological data. When instructed, the electrode patch requests a reading from the physiological sensor according to programmed timing. Alternatively, the physiological sensors are provided as part of the circuitry of the monitor recorder and each electrode patch is programmed to provide sampling instructions from the physiological sensor. For instance, some physiological data should be collected more frequently than other types of physiological data. In a further embodiment, the electrode patch 15 can store sampling instructions for more than one physiological sensor at a time.

In one example, a high resolution temperature sensor can be provided on the electrode patch or as part of the circuitry of the monitor recorder to take temperature measures at a high sample rate. Instructions from the electrode patch, which have been preprogrammed, instruct the high resolution temperature sensor when to begin measuring temperature and when to stop measuring the temperature, as well as how many times the temperature should be measured over a time period, which allows the electrocardiography and physiological sensor monitor to obtain the data and also continually monitor the ECG signals over time without utilizing large amounts of processing power and memory of the monitor. The instructions can also include a duration of measurement, which can be the same or different for each obtained data measurement.

In a further embodiment, multiple patches, each programmed for a different physiological sensor or with different sampling rates for the same physiological sensor can be used with the same monitor recorder.

While the invention has been particularly shown and described as referenced to the embodiments thereof, those skilled in the art will understand that the foregoing and other changes in form and detail may be made therein without departing from the spirit and scope.

### CLAUSES

1. An extended wear electrocardiography patch (15), comprising:
   a flexible backing (20) formed of an elongated strip (21) of stretchable material;
   an electrocardiographic electrode (38, 39) respectively affixed to and
   conductively exposed on a contact surface of each end of the elongated strip (21);
   a flexible circuit (32) affixed on each end to the elongated strip (21)
   and comprising a pair of circuit traces (33, 37) each originating within one of 9 the ends of the elongated strip (21) and electrically coupled to one of the electrocardiographic electrodes (38, 39);
   a non-conductive receptacle (25) securely adhered on the one end of the elongated strip (21) opposite the contact surface and formed to removably
   receive an electrocardiography monitor (14) operable to obtain
   electrocardiographic signals through the electrocardiographic electrodes (38,15 39), wherein the non-conductive receptacle (25) comprises electrode terminals aligned to electrically interface the pair of circuit traces (33, 37) to the electrocardiography monitor (14); and
   a crypto circuit (103) comprising memory programed with a sampling rate for at least one physiological sensor provided at least one of with the electrocardiography monitor (14) and on the flexible backing (20) to instruct 21 the physiological sensor to obtain readings of physiological data.
2. An extended wear electrocardiography patch (15) according to Clause 1, further comprising:
   a download station (125) configured to retrieve the electrocardiographic signals.
3. An extended wear electrocardiography patch (15) according to Clause 2, further comprising:
   middlewear operable to convert the electrocardiographic signals into a different format.
4. An extended wear electrocardiography patch (15) according to Clause 3, further comprising:
   a server to remotely interface with the download station (125) and configured to retrieve the formatted electrocardiographic signals.
5. An extended wear electrocardiography patch (15) according to Clause 1, wherein the physiological sensor comprises one of a SpCh sensor, blood pressure sensor, temperature sensor, respiratory rate sensor, glucose sensor, airflow sensor, and volumetric pressure sensor.
6. An extended wear electrocardiography patch (15) according to Clause 1, wherein the electrocardiography monitor (14) comprises:
   a sealed housing adapted to be removably secured into the non- conductive; and
   electronic circuity comprised within the sealed housing, comprising: an electrocardiographic front end circuit operable to sense the electrocardiographic signals through the electrocardiographic electrodes (38, 39) provided on the elongated strip (21); and
   flash memory to record the electrocardiographic signals.
7. An extended wear electrocardiography patch (15) according to Clause 1, wherein the electronic circuitry of the electrocardiography monitor (14) is configured to embed the physiological data into a stream of the electrocardiographic signals.
8. An extended wear electrocardiography patch (15) according to Clause 1, wherein the physiological data is matched to the electrocardiographic signals.
9. An extended wear electrocardiography patch (15) according to Clause 1, wherein the physiological sensor records the physiological data based on the sampling rate.
10. An extended wear electrocardiography patch (15) according to Clause 1, further comprising: a battery (79) to provide power to the physiological sensor that is electrically interfaced with the micro-controller over the expansion bus.
11. An extended wear electrocardiography and physiological sensor monitor (12), comprising:
   an electrocardiography monitor (14), comprising:
   a sealed housing; and
   electronic circuitry comprised within the sealed housing, comprising:
      an externally-powered micro-controller; and an electrocardiographic front end circuit electrically interfaced to the micro-controller and operable to sense electrocardiographic signals; and
      an electrocardiography patch (15), comprising:
         a flexible backing (20) formed of an elongated strip (21) of stretchable material;
         an electrocardiographic electrode (38, 39) affixed to and conductively exposed on a contact surface of each end of the elongated strip (21);
         a flexible circuit (32) affixed on each end to the elongated strip (21) and comprising a pair of circuit traces (33, 37) each originating within one of the ends of the elongated strip (21) and electrically coupled to one of the electrocardiographic electrodes (38, 39);
         a non-conductive receptacle (25) securely adhered on the one end of the elongated strip (21) opposite the contact surface and formed to removably receive the electrocardiography monitor (14), the non-conductive receptacle (25) comprising electrode terminals aligned to electrically interface the pair of circuit traces (33, 37) to the electrocardiography monitor (14); and a crypto circuit (103) comprising memory programed with a sampling rate for at least one physiological sensor provided at least one of with the electrocardiography monitor (14) and on the flexible backing (20) to instruct the physiological sensor to obtain readings of physiological data.
12. An extended wear electrocardiography and physiological sensor monitor (12) according to Clause 11, further comprising:
   a download station (125) configured to retrieve the
   electrocardiographic signals.
13. An extended wear electrocardiography and physiological sensor monitor (12) according to Clause 12, further comprising:
   middlewear operable to convert the electrocardiographic signals into a different format.
14. An extended wear electrocardiography and physiological sensor monitor (12) according to Clause 13, further comprising:
   a server to remotely interface with the download station (125) and configured to retrieve the formatted electrocardiographic signals.
15. An extended wear electrocardiography and physiological sensor monitor (12) according to Clause 11, wherein each physiological sensor comprises one of a SpCh sensor, blood pressure sensor, temperature sensor, respiratory rate sensor, glucose sensor, airflow sensor, and volumetric pressure sensor.
16. An extended wear electrocardiography and physiological sensor monitor (12) according to Clause 11, wherein the electronic circuitry is configured to embed the physiological data into a stream of the
   electrocardiographic signals.
17. An extended wear electrocardiography and physiological sensor monitor (12) according to Clause 11, wherein the physiological data is matched to the electrocardiographic signals.
18. An extended wear electrocardiography and physiological sensor monitor (12) according to Clause 11, further comprising:
   at least one other electrocardiography patch (15) to replace the electrocardiography patch (15), wherein each electrocardiography patch (15) is specialized to provide a different sampling rate for different physiological data.
19. An extended wear electrocardiography and physiological sensor monitor (12) according to Clause 11, wherein the physiological sensor records the physiological data based on the sampling rate.
20. An extended wear electrocardiography and physiological sensor monitor (12) according to Clause 11, further comprising:
   a battery (79) from which the physiological sensor draws power, wherein the physiological sensor is electrically interfaced with the micro- controller over an expansion bus; and
   flash memory of the electrocardiography monitor (14) operable through the expansion bus to store samples of the physiological data sensed by the physiological sensor.

## Claims

1. A moisture-resistant electrocardiography monitor, comprising:
an electrocardiography monitor recorder, comprising:
a wearable housing molded out of one or more materials and sealed against moisture;
a plurality of electrical contacts protruding from the wearable housing;
a seal coupling positioned on the wearable housing and surrounding the electrical contacts; and
electronic circuitry provided within the wearable housing, the electronic circuitry further comprising:
an electrocardiographic front end circuit under a control of a micro-controller and adapted to sense cardiac electrical potential differentials through electrocardiographic electrodes via some of the electrical contacts, which are provided to the micro-controller as an analog signal;
the micro-controller configured to sample the analog signal; and
a memory electrically interfaced with the micro-controller and operable to store the samples; and
an extended wear electrode patch, comprising:
a flexible backing comprising a plurality of adhesive contact surfaces;
the electrocardiographic electrodes, each comprised on one of the adhesive contact surfaces;
a receptacle affixed to a non-contacting surface of the flexible backing into which the wearable housing can be removably secured, the receptacle comprising a compartment within which a component interfaced to the electronic circuitry is positioned via some of the electrical contacts, wherein the component is a battery;
a plurality of electrical pads positioned on the receptacle, each of the pads configured to interface with one of the electrical contacts when the wearable housing is secured within the receptacle; and
a moisture-resistant seal formed on the receptacle and surrounding the electrical pads, wherein the moisture-resistant seal mates the seal coupling when the wearable housing is secured within the receptacle.

2. A monitor according to claim 1, wherein the one or more materials comprise at least one of polycarbonate and ABS.

3. A monitor according to claim 1, wherein the one or more materials comprise an alloy of polycarbonate and ABS.

4. A monitor according to claim 1, the housing further comprising a waterproof patient-operable tactile-feedback button.

5. A monitor according to claim 4, wherein an outer surface of the button is molded out of a soft pliable material.

6. A monitor according to claim 5, wherein the soft pliable material comprises silicon rubber.

7. A monitor according to claim 1, wherein the seal coupling and the moisture resistant seal are circular.

8. A monitor according to claim 1, wherein the battery is replaceable without opening the wearable housing.

9. A monitor according to claim 1, wherein the compartment is formed on a bottom surface of the receptacle.

10. A monitor according to any one of claims 1 to 9, wherein the monitor is patient-interfacing, the electrocardiography monitor recorder further comprises a waterproof patient-operable tactile feedback button positioned on an outside of the wearable housing, and the electronic circuitry is powered by the battery via at least some of the electrical contacts.

11. A monitor according to claim 10, wherein the waterproof patient-operable tactile feedback button is positioned on a side of the housing opposite to a further side on which the electrical contacts are positioned.

12. A monitor according to claim 10, wherein the battery is one of a replaceable, rechargeable, and disposable battery.
